# EUROPEAN PATENT APPLICATION

(11) **EP 4 000 693 A1**
(43) Date of publication of application: **25.05.2022**
(21) Application number: 20207972.9
(22) Date of filing: 17.11.2020
(51) Int. Cl.: A61Q 17/04, A61K 8/86, A61Q 19/00

(54) **TWO COMPONENT MOISTURIZER**

(71) Applicant: Beiersdorf AG, 20253 Hamburg (DE)
(72) Inventor: Ferraro, Claudio, 1007 Lausanne (CH); Köhler, Manuele, 22527 Hamburg (DE)
(74) Representative: Beiersdorf AG

(57) **Abstract**

The present invention belongs to the cosmetic field.

## Description

The present invention belongs to the cosmetic field and relates to a two component skin moisturizing kit and the use thereof.

A beautiful and attractive appearance is a desire for many people. One typical sign of such an appearance is a healthy and smooth looking skin. Therefore, in order to take care on the skin, it is for many people a daily routine to apply cosmetic products such as body lotions and sunscreen compositions.

Commonly cosmetic products are used to make the consumer feel better in their skin. Thereby, one aspect is directed to the fact that cosmetic products are often used to moisturize the skin in order to avoid dry appearance of the skin. For that purpose so called moisturizer are contained in the products. Moisturizers are cosmetic ingredients that are intended to hydrate the skin. They also increase the water content of the skin, giving it a smooth appearance and provide a barrier against the loss of water from the skin.

Typical moisturizers contained in almost all cosmetic products are glycerol, propylene glycol and sorbitol.

The ability to moisturize the skin can be measured using a corneometer. For that purpose the corneometer value before and after application of the cosmetic product is compared. The higher the corneometer value, the better is the moisturization.

However, even though the principle of moisturizing the skin is quite common, the ability of the cosmetic products to moisturize the skin is still limited. Accordingly, there is a general need of products, methods and applications, which allow the moisturization capabilities to be increased. In particular it is desirable if a better moisturiziation is achieved for a period of at least 20 hours.

It was now surprisingly found that by applying a polymeric primer in a first step and applying a conventional cosmetic product containing at least one moisturizer in a second step, a significant improved moisturization of the skin can be achieved.

The synthesis of polymers falling in the scope of the invention are disclosed in the article *"*Amphibious superamphiphilic fabrics with self-healing underwater superoleophilicity", (Mater. Horiz., 2019, 6, 122). However, the article does not disclose cosmetic compositions at all.

It was now surprisingly found by the applicant that the disadvantages discussed above can be addressed by the present invention.

The present invention is a method of moisturizing the mammal skin involving the steps of
a) applying a first cosmetic composition A comprising a polymer polymerized from a monomer mixture comprising
   (i) at least one triglycidylether according to one of the formulas (I and II), 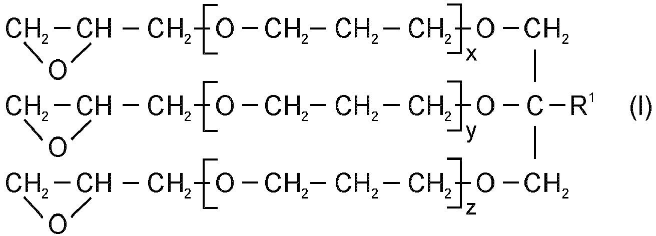 and 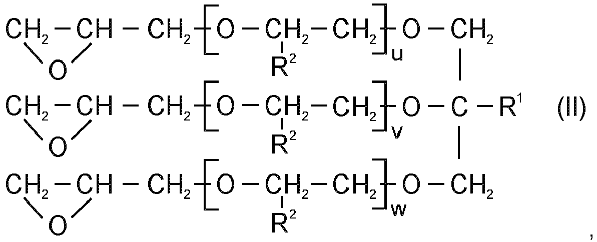 wherein
      R¹ stands independently for an alkylgroup having 1 to 4 carbon atoms or a hydrogen atom,
      R² stands independently for a linear or branched, saturated or non-saturated hydrocarbon group containing 1 to 18 carbon atoms or a hydrogen atom, and
      u, v, w, x, y and z stand independently for an integer number in the range from 1 to 100;
         and
   (ii) at least one alkylamine comprising 3 to 30 carbon atoms;
   and
b) applying a second cosmetic composition B comprising at least one moisturizer.

A second aspect of the invention is the use of a polymer polymerized from a monomer mixture comprising
(i) at least one triglycidylether according to one of the formulas I to II,
   wherein R¹, R², u, v, w, x, y and z are defined as described above,
   and
(ii) at least one alkylamine comprising 3 to 30 carbon atoms;
to form a coating on the mammal skin, which allows the moisturization of the mamal skin to be increased if a moisturizer is applied onto the coating.

Moisturizer are understood to be all substances which are listed in the International Cosmetic Ingredient Dictionary and Handbook, Thirteenth Edition 2010, (ISBN 1-882621-47-6) under the function "humectant".

A third aspect of the invention is a cosmetic composition A comprising at least one polymer polymerized from a monomer mixture comprising
(i) at least one triglycidylether according to one of the formulas I to II,
   wherein R¹, R², u, v, w, x, y and z are defined as described above,
   and
(ii) at least one alkylamine comprising 3 to 30 carbon atoms.

A forth aspect of the invention is a kit comprising the cosmetic composition A and a second cosmetic composition B, wherein the second composition comprises at least one moisturizer.

A fifth aspect of the invention is a polymer polymerized from a monomer mixture comprising
(i) at least one triglycidylether according to one of the formulas I to II, wherein R¹, R², u, v, w, x, y and z are defined as described above,
   and
(ii) at least one alkylamine comprising 3 to 30 carbon atoms.

It was surprisingly noticed that the invention allows to moisturize the skin synergistically. Accordingly, the objectives of the present invention are solved.

All the weight percentages (% by weight) given below relate, unless otherwise stated, to the total weight of the cosmetic composition A.

If ratios of certain components are disclosed in the following description, these ratios refer, unless otherwise stated, to weight ratios of the components.

Unless otherwise stated, all tests and measurements were performed under "normal conditions". The term "normal conditions" refers to 20°C, 1013 hPa and a relative humidity of 50%.

In the following description the terms "according to the invention", "preferred according to the invention" and so on are always directed to the use according to the invention, to the method according to the invention and to the cosmetic composition according to the invention.

For the purposes of the present disclosure, the term "free from" means that the proportion of the respective polymer is less than 0.05% by weight. This ensures that entrainments or impurities with these polymers are not included as "free from" according to the invention.

The term "skin" refers to the mammal skin, preferably to the human skin.

Emulsifiers are understood to be all substances which are listed in the International Cosmetic Ingredient Dictionary and Handbook, Thirteenth Edition 2010, (ISBN 1-882621-47-6) under the name "emulsifying agent". Surfactants are understood to be all substances which are listed in the International Cosmetic Ingredient Dictionary and Handbook, Thirteenth Edition 2010, (ISBN 1-882621-47-6) under the name "surfactant".

Without being bound by the theory it is believed that the amine reacts with the epoxide groups of the triglycidylether allowing a structured network to be created. Alternatively, the amine allows for nucleophilic substitution at the carbon atom with the OH group or the Nu group. It should be noted that every amine may react with up to two triglycidylether such that extensive crosslinking occurs, allowing not a clear chemical structure to be defined.

It is preferred according to the invention if the polymer is polymerized from a monomer mixture comprising
(i) 10% to 90% by weight of at least one triglycidylether according to one of the formulas I to II, wherein R¹, R², u, v, w, x, y and z are defined as described above, and
(ii) 10% to 90% by weight of at least one alkylamine comprising 3 to 30 carbon atoms.

It is further preferred according to the invention if the polymer is polymerized from a monomer mixture comprising
(i) 50% to 90% by weight of at least one triglycidylether according to one of the formulas I to II, wherein R¹, R², u, v, w, x, y and z are defined as described above, and
(ii) 10% to 50% by weight of at least one alkylamine comprising 3 to 30 carbon atoms.

It is more preferred according to the invention if the polymer is polymerized from a monomer mixture comprising
(i) 50% to 90% by weight of at least one triglycidylether according to one of the formulas I to II, wherein R¹, R², u, v, w, x, y and z are defined as described above, and
(ii) 10% to 50% by weight of at least one alkylamine comprising 3 to 30 carbon atoms.

It is most preferred according to the invention if the polymer is polymerized from a monomer mixture comprising
(i) 75% to 88% by weight of at least one triglycidylether according to one of the formulas I to II, wherein R¹, R², u, v, w, x, y and z are defined as described above, and
(ii) 12% to 25% by weight of at least one alkylamine comprising 3 to 30 carbon atoms.

For the case that the polymer is polymerized from a monomer mixture comprising at least one triglycidylether according to formula I, it is preferred if R¹ is a hydrogen atom. It is further preferred if the sum of the integer values of x, y and z is less than 20, more preferred less than 10 and most preferred less than 6. It is most preferred if x, y and z are all equal to 1.

For the case that the polymer is polymerized from a monomer mixture comprising at least one triglycidylether according to formula II, it is preferred if R¹ is a hydrogen atom. It is further preferred if R² stands for a methyl group and/or a hydrogen atom, whereby it is most preferred if R² stands for a methyl group. It is further preferred if the sum of the integer values of u, v and w is less than 20, more preferred less than 10 and most preferred less than 6. It is most preferred if u, v and w are all equal to 1.

Furthermore, it is preferred if the amine is a primary amine. It is further preferred if the amine comprises a linear alkyl chain comprising 10 to 25 carbon atoms, more preferably 14 to 22 and most preferred 18 carbon atoms.

The polymer of the invention is preferably formed by
a) dissolving the triglycidylether accoding to the invention in ethanol,
b) then adding the amine to the solution, and
c) stirring the obtained mixture to obtain the product.

It is preferred if the ratio by weight between the triglycidylether accoding to the invention and ethanol is in the range from 1:1 to 1:100, more preferably from 1:2 to 1:50, more preferably from 1:5 to 1:20, and most preferred from 1:9 to 1:11.

It is further preferred if the mixture is stirred at 50°C for at least 5 minutes. Most preferably for 12 to 20 minutes.

If desired, ethanol may be removed from the reaction product.

In one further aspect of the present invention it is further preferred if the reaction product obtained in step c) is further reacted in a further step d) with another nucleophile in order to ensure that all epoxide groups are opened and no closed epoxide rings remain in the polymeric structure. Preferred nucleophiles may be selected from the group of water, alcohols, amines, hydrohalic acids, thiols and/or phosphorous acids. Most preferred nucleophils are water, hydrochloric acid, and alcohols.

It is particular preferred if the polymers formed according to the invention are characterized in that they have an average molecular weight in the range from 1000 to 1000000 Dalton, more preferably in the range from 3000 to 100000 Dalton and most preferably 4000 to 10000 Dalton.

According to the invention the average molecular weight of the polymers according to the invention is determined by GPC analysis using a reference material, eg. Poly(styrene) standards.

It is further preferred, if the cosmetic composition A, which is according to the invention, comprises the polymer according to the invention in a total quantity ranging from 0.5 to 30% by weight, more preferably 1 to 20% by weight and most preferably from 5 to 18% by weight, calculated to the total weight of the composition A.

Composition A is further preferably characterized in that in comprises ethanol. If ethanol is contained, the total quantity of ethanol is preferably in the range from 15 to 95% by weight, more preferably from 50 to 90% by weight and most preferably from 60 to 80% by weight, calculated to the total weight of the composition A.

Moreover, it is further preferred if the composition A comprises water. If water is contained, the total quantity of water is preferably in the range from 5 to 95% by weight, more preferably from 15 to 80% by weight and most preferably from 20 to 70% by weight, calculated to the total weight of the composition A.

Additionally, it is further preferred if the composition A comprises glycerol. If glycerol is contained, the total quantity of glycerol is preferably in the range from 1 to 30% by weight, more preferably from 2 to 20% by weight and most preferably from 2 to 10% by weight, calculated to the total weight of the composition A.

In another embodiment it is preferred if the composition A does not comprise glycerol.

Furthermore, it is preferred, if composition A comprises propylene glycol, whereby it is further preferred if the total quantity of propylene glycol is in the range from 1 to 30% by weight, calculated to the total weight of the composition A.

In another embodiment it is preferred if the composition A does not comprise propylene glycol.

In still another embodiment it is preferred if the composition A does not comprise a moisturizer.

In further embodiment the composition A may further comprise additional ingredients commonly contained in cosmetic compositions.

For example, it is preferred if composition A further comprises ethylhexylglycerin, hexandiol, and/or phenoxyethanol.

For the case that ethylhexylglycerin is contained it is preferred if the total quantity of ethylhexylglycerin is in the range from 0.1 to 3% by weight, calculated to the total weight of composition A.

For the case that hexandiol is contained it is preferred if the total quantity of hexandiol is in the range from 0.1 to 3% by weight, calculated to the total weight of composition A.

For the case that phenoxyethanol is contained it is preferred if the total quantity of phenoxyethanol is in the range from 0.1 to 3% by weight, calculated to the total weight of composition A.

In one aspect of the present invention the composition A is characterized in that no UV-filter is contained.

In another aspect of the present invention the composition A comprises at least one water-soluble UV filter. Embodiments of the present invention which are advantageous according to the invention are characterized in that the total amount of water-soluble UV filters in the preparation is from 0.1 to 18% by weight, based on the total weight of the composition A.

It is advantageous according to the invention if sulfonic acid salts are used as water-soluble UV filters. Preferred examples are 1,4-di (2-oxo-10-sulfo-3-bomylidenemethyl) benzene and its salts; 4-(2-oxo-3-bornylidenemethyl) benzenesulfonic acid; 2-methyl-5- (2-oxo-3-bornylidenemethyl) sulfonic acid salts; Terephthalidendicamphersulfonsäure; Compounds 2-phenylbenzimidazole-5-sulfonic acid and / or salts thereof and / or phenylene-1,4-bis (2-benzimidazyl) -3,3'-5,5'-tetrasulfonic acid salts.

The counterion of the salts is usually sodium, potassium or ammonium ions, the sodium salts being preferred according to the invention.

According to the invention, the water-soluble UV filters are preferably selected from the group of the compounds 2-phenylbenzimidazole-5-sulfonic acid and / or salts thereof and / or phenylene-1,4-bis (2-benzimidazyl) -3,3'-5 , 5'-tetrasulfonsäuresalze. The use of 2-phenylbenzimidazole-5-sulfonic acid and / or salts thereof is particularly preferred according to the invention.

The preferred use concentration for 2-phenylbenzimidazole-5-sulfonic acid and / or salts thereof is from 0.1 to 12% by weight, based on the total weight of the composition A.

Furthermore, it is still possible and preferred if composition A comprises at least one additional UV-filter, which may preferably be selected from the group consisting of 2,2'-methylene-bis- (6- (2H-benzotriazol-2-yl) -4- (1 , 1, 3,3-tetramethylbutyl) phenol); 2- (2H-Benzotriazol-2-yl) -4-methyl-6- [2-methyl-3- [1,3,3,3-tetramethyl-1- [(trimethylsilyl) oxy] disiloxanyl] propyl] phenol; 3- (4-methylbenzylidene) camphor; 3-benzylidenecamphor; ethylhexyl salicylate; 2-ethylhexyl-2-cyano-3,3-diphenylacrylate; 4- (dimethylamino) benzoic acid (2-ethylhexyl) ester; 4- (dimethylamino) benzoic acid amyl ester; 4-Methoxybenzalmalon-säuredi (2-ethylhexyl) ester; 4-methoxycinnamate (2-ethylhexyl) ester; Isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl; 2-ethylhexyl 2-hydroxybenzoate; dimethicodiethylbenzalmalonate; 3- (4- (2,2-ethoxycarbonylvinyl) phenoxy) propenyl) methoxysiloxane / dimethylsiloxane copolymer; 4- (tert-butyl) -4'-methoxydibenzoylmethane; Hexyl 2- (4'-diethylamino-2'-hydroxybenzoyl) benzoate; dioctylbutylamidotriazone (INCI: diethylhexyl-butamidotriazone); 2,4-bis-[5-1 (dimethylpropyl) benzoxazol-2-yl- (4-phenyl) imino] -6- (2-ethylhexyl) -imino-1,3,5-triazine having the (CAS No 288254-16-0); 2,4-Bis-{[4-(2-ethylhexyloxy)-2-hydroxy]-phenyl}-6-(4-methoxyphenyl)-1, 3,5-triazine (INCI: bis-ethylhexyloxyphenol methoxyphenyl triazine) ; 4,4',4 "-(1,3,5-triazine-2,4,6-triyltriimino) tris-benzoic acid tris (2-ethylhexyl ester) (also: 2,4,6-tris [anilino] (p-carbo-2'-ethyl-1'-hexyloxy)]-1,3,5-triazine (INCI: ethylhexyl triazone); 2, 4, 6-tribiphenyl-4-yl-1,3,5-triazine merocyanines; titanium dioxide and zinc oxide.

Although, for the case that the composition A contains at least one moisturizer, it is already possible to enhanced the moisturization by applying composition A, it was surprisingly noticed that the optimum skin moisturization may be achieved if composition A is applied to the skin in a first step and in a second step a conventional cosmetic composition comprising a moisturizer, herein named as composition B, is applied.

It is an advantage, if the composition B is an emulsion. Emulsions, here in particular W/O, O/W or W/O/W emulsions, are often used as cosmetic or medical preparations. Emulsions are generally understood as meaning heterogeneous systems which comprise two liquids which are immiscible or only miscible with one another to a limited extent, which liquids are normally referred to as phases. In an emulsion, one of the two liquids is dispersed in the other liquid in the form of very fine droplets.

If the two liquids are water and oil and if oil droplets are finely distributed in water, then this is an oil in water emulsion (O/W emulsion, e.g., milk). The basic character of an O/W emulsion is determined by the water. In the case of a water-in-oil emulsion (W/O emulsion, e.g., butter), the principle is reversed, the basic character here being determined by the oil.

Alternatively, composition B is a single phase alcohol (ethanol) containing formulation. High quantities of ethanol are used in this kind of formulations in order to provide a clear solution of all ingredients. The amount is ethanol is chosen to be sufficient to form a stable and transparent single phase formulation.

In accordance with the present invention the composition B is preferably characterized in that it is an O/W emulsion, a W/O emulsion or a single phase alcoholic solution.

Composition B comprises at least one moisturizer.

Preferred moisturizer to be contained in the composition B are selected from the group of glycerol, propylene glycol and sorbitol.

According to the invention it is preferred if the total quantity of the moisturizers, especially of glycerol, propylene glycol and/or sorbitol is in the range from 0.1 to 30% by weight, more preferably from 0.5 to 25% by weight and most preferably from 2 to 20% by weight, calculated to the total weight of composition B.

In addition composition B may comprise further ingredients conventionally used in cosmetic compositions.

Composition B according to the invention is advantageously characterized in that the composition comprises one or more organic UV-A filters selected from the group of the compounds 4- (tert-butyl) -4'-methoxydibenzoylmethane and 2- (4'-diethylamino-2 ' - hydroxybenzoyl) -benzoic acid hexyl ester.

It is inventively preferred if the organic UV-A filter compound 4- (tert-butyl) -4'-methoxydibenzoylmethane is used in composition B.

The advantageous total use according to the invention for the organic UV-A filter, in particular 4- (tert-butyl) -4'-methoxydibenzoylmethane and/or 2- (4'-diethylamino-2'-hydroxybenzoyl) - benzoic acid hexyl ester, is/are from 1 to 5 weight -%, preferably 1.5 to 4.5% by weight, particularly preferably 2 to 4% by weight, based on the total weight of the composition B.

Embodiments of the present invention which are advantageous according to the invention are characterized in that the composition B contains one or more organic UV filters selected from the group of the compounds 2-phenylbenzimidazole-5-sulphonic acid and/or salts thereof; phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; terephthalidenedicamphorsulphonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bisethoxycarbonylvinyl)phenoxy)propenyl)methoxysiloxane/dimethylsiloxan e copolymer; dioctylbutylamidotriazone (INCI: Diethylhexyl Butamido Triazone); 2,4-bis[5-1 (dimethylpropyl)benzoxazol-2-yl(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; and merocyanine.

It is inventively preferred if the composition B contains 2-phenylbenzimidazole-5-sulfonic acid and / or salts thereof. For the case that the composition B contains 2-phenylbenzimidazole-5-sulfonic acid and / or salts the total quantity of those substances is preferably in the range from 0.5 to 5% by weight, more preferably 0.6 to 4.5% by weight and most preferably 0.8 to 4% by weight, based on the total weight of the composition B.

It is further advantageous if the composition B comprises 2-ethylhexyl-2-cyano-3,3-diphenylacrylate (octocrylene), whereby it is further preferred if the total quantity of this substance is in the range from 1 to 12% by weight, preferably 1.5 to 6% by weight, particularly preferably 2 to 3.5% by weight based on the total weight of composition B.

It is further preferred if the composition B comprises Homosalate in a total quantity from 1 to 12% by weight, preferably 1.5 to 6% by weight, particularly preferably 2 to 3.5% by weight based on the total weight of composition B.

It is further preferred if the composition B comprises Bis-Ethylhexyloxyphenol Methoxyphenyl Triazine in a total quantity from 0.5 to 12% by weight, preferably 0.6 to 6% by weight, particularly preferably 0.7 to 2.5% by weight based on the total weight of composition B.

It is further preferred if the composition B comprises Ethylhexyl Salicylate in a total quantity from 0.5 to 12% by weight, preferably 2 to 9% by weight, particularly preferably 3 to 7.5% by weight based on the total weight of composition B.

It is further preferred if the composition B comprises Silica Dimethyl Silylate in a total quantity from 0.1 to 2% by weight, preferably 0.4 to 1.5% by weight, particularly preferably 0.6 to 1.3% by weight based on the total weight of composition B.

Moreover, the composition B of the present invention is preferably characterized in that at least one inorganic UV-filter is contained, especially zinc oxide and/or titanium dioxide. It is especially preferred, if the total quantity of zinc oxide and/or titanium dioxide in the composition B of the present invention is in the range from 0.1 to 5% by weight, calculated to the total weight of the composition B.

For the case that composition B is an emulsion, it is preferred if the total quantity of the oil phase in the composition B is in the range from 0.9 to 25% by weight, more preferably 1 to 20% by weight, more preferably 1.1 to 19% by weight and most preferably from 1.2 to 18% by weight, calculated to the total weight of the composition B.

For the case that composition B is an emulsion, it is preferred if the ratio of the total quantity of the emulsifier to the total quantity of the oil phase is in the range from 1:1 to 1:7, more preferably 1:1.1 to 1:4, more preferably 1:1.2 to 1:3 and most preferably 1:1.4 to 1:2.8. It is noted that by definition emulsifier and surfactants are not calculated to the total quantity of the oil phase.

According to the invention, it is further preferred if the composition B contains phenoxyethanol. In the case the composition B contains phenoxyethanol the total quantity of phenoxyethanol is preferably in the range from 0.1 % by weight to 2% by weight and more preferably from 0.4% by weight to 1% by weight, calculated to the total weight of the composition B.

Moreover, preferred composition B of the present invention are therein characterized that they contain ethylhexylglycerin, whereby it is further preferred if the total quantity of ethylhexylglycerin is in the range from 0.05% to 0.5% by weight, calculated to the total weight of the composition B.

Furthermore, it is preferred if the composition B does not contain an alkylparabene such as propyl parabene and/or butyl parabene.

According to the invention it is further preferred if the composition B comprises at least one polymeric rheology modifier. The term "polymeric rheology modifier" is understood as a polymer which increases the viscosity of the composition B if added to it. Preferred are the polymeric rheology modifier selected from the group carbomer, xanthan gum and hydroxyethylcellulose. Most preferred are carbomer and/or xanthan gum.

The total quantity of the polymeric rheology modifier is preferably in the range from 0.01 to 0.8% by weight, more preferably 0.05 to 0.5% by weight and most preferably 0.15 to 0.4% by weight, calculated to the total weight of the composition B.

In addition, preferred compositions B are characterized in that they contain ethanol. In the case that ethanol is contained in the emulsions according to the invention, it if further preferred if the total quantity of ethanol is in the range from 2% by weight to 6% by weight, calculated to the total weight of the composition B.

Further, it is preferred if the composition B comprises glycerol, whereby it is further preferred if the total quantity of glycerol is at least 2% by weight, more preferably at least 2.5% by weight, more preferably at least 3% by weight, more preferably at least 5% by weight, more preferably at least 6% by weight, more preferably at least 7% by weight, more preferably at least 8% by weight, more preferably at least 9% by weight, more preferably at least 10% by weight, more preferably at least 11% by weight, more preferably at least 12% by weight; and whereby the total quantity of glycerol should not exceed 14% by weight, calculated to the total weight of the composition B.

Preferred embodiments of the invention are further characterized in that the composition B comprises at least cetyl alcohol, stearyl alcohol and/or cetearyl alcohol. In the case cetyl alcohol, stearyl alcohol and/or cetearyl alcohol are contained, the total quantity of those substance is preferably in the range from 0.5 to 8% by weight, more preferably from 1 to 6% by weight, calculated to the total weight of the composition B.

Additionally, it is preferred according to the invention if the total quantity of water in composition B is in the range from 45% to 95% by weight, more preferably from 48% to 70% by weight and most preferably from 52% to 65% by weight, calculated to the total weight of the composition B.

According to the invention the composition B may preferably contain at least one compound selected from the group of limonene, linalool, citral, alpha-isomethylionone and geraniol.

Embodiments of the present invention which are advantageous according to the invention are characterized in that the composition B contains as further ingredients one or more compounds selected from the group of the compounds alpha-lipoic acid, folic acid, phytoene, D-biotin, coenzyme Q10, alpha-glucosylrutin, carnitine, carnosine, natural and / or synthetic isoflavonoids, flavonoids, creatine, creatinine, taurine, β-alanine, tocopheryl acetate, dihydroxyacetone, glycyrrhetinic acid, 8-hexadecene-1, 16-dicarboxylic acid, glycerylglycose, (2-hydroxyethyl) urea and / or licochalcone A.

According to the invention it is further preferred if the composition B has a pH value in the range from 4.5 to 8.

### Examples:

The following examples should illustrate the compositions of this invention, without intending to limit the invention to these examples. The numerical values in the examples are percentages by weight, based on the total weight of the compositions.

The polymer of the invention was synthesized in the following manner:
5 g of Glycerol propoxylate triglycidyl ether (GPTE) (formula II, wherein R¹ is an hydrogen, R² is a methyl group and *u, v* and w are each equal to 1, was added to 50 ml ethanol and then stirred for 30 min. 1 g of Octadecylamine (ODA) was added to the as-prepared solution. The solution was then further stirred for 15 min at 50 °C.

The obtained polymer had an average molecular weight of 5220 Da, determined using GPC analysis with Polystyrene as calibration standard. The resulting molecule was further analyzed and the structure was confirmed, as also described in the article *"*Amphibious superamphiphilic fabrics with self-healing underwater superoleophilicity", (Mater. Horiz., 2019, 6, 122).

In the reaction process a nucleophilic substitution occurs, wherein amine group of the alkylamine attacks the electrophilic C of the C-O bond of the epoxide group causing it to break, resulting in ring opening. This reaction may occur multiple times resulting in a continuous polymerization network.

For the following experiments, the output of the procedure described above was used under the designation polymer blend.

The following composition A was used in the experiments:

| Ingredient | Precoating 1 |
|---|---|
| Alcohol Denat. | 88 |
| Polymer blend | 12 |

A conventional care cream was used as composition B, which is commercially available under the name "Nivea Care". This preparation contains glycerine as a moisturizer. The product has the following INCI list:
Aqua, Glycerin, Butyrospermum Parkii Butter, Cetyl Palmitate, Olus Oil, Cetyl Alcohol, Isopropyl Palmitate, Dimethicone, Sodium Polyacrylate, Phenoxyethanol, Linalool, Citronellol, Alpha-Isomethyl lonone, Geraniol, Limonene, Parfum

The experiments determining the moisturization capabilities of the invention were carried out in the following manner:
The skin moisture was measured by means of capacitance measurements of a corneometer (Corneometer CM 825 from Courage + Khazaka, Cologne, Germany). All measurements were made at a room temperature of 21 ° C and a relative humidity of 50%.

A section of pig skin was cleaned and used as test side for the measurements. A reference measurement t0 (0h) was carried out on each test side before applying a composition.

Four test sides were treated. The first was simply treated with ethanol. The second was simply treated with composition A. The third section was first treated with composition B. The fourth test side was treated with composition A first, followed by an application of composition B. On each test side a total amount of 2mg/cm² corresponding to 52.8 mg on 26.4 cm² of each composition named above was applied.

The resulting corneometer values are presented in the table below:

| Test side/ time after application | 0h | 1h | 2h | 3h | 4.5h | 6.5h | 7h |
|---|---|---|---|---|---|---|---|
| Ethanol | 18.2 | 22.6 | 24.2 | 23.7 | 22.3 | 19.1 | 21.6 |
| Composition A | 26.7 | 33.5 | 29.7 | 29.5 | 28.7 | 26.8 | 27.0 |
| Composition B | 24.3 | 36.0 | 33.6 | 34.6 | 32.3 | 28.0 | 30.5 |
| Composition A + Composition B | 20.7 | 38.4 | 35.8 | 36.1 | 34.0 | 32.5 | 33.0 |

Thus, it was found by applying composition A first and adding composition B second a superior skin moisturization was achieved.

Further synthesis were performed:
5 g of Glycerol propoxylate triglycidyl ether (GPTE) according to formula I, wherein R¹ is a hydrogen atom and *x*,*y* and z are each equal to 1, were added to 50 ml ethanol and then stirred for 30 min. 1 g of Octadecylamine (ODA) was added to the as-prepared solution. The solution was then further stirred for 15 min at 50 °C to obtain the final product.

The reaction products obtained in both reactions were one time further reacted in order to open the remaining epoxide groups. In order to do that Water was added to the reaction solution then pH of the reaction mixture was modified step-wise by addition of 1M HCI to reach pH 2. Via ¹H-NMR in CDCl₃ at 300 MHz the change in contained number of epoxide groups was monitored. Before adding HCI the ratio of epoxide group (2.7 ppm) and the partial alkane group (4.1 -3.2 ppm) was 1:44. After addition to reach pH 2 the ratio was 1: 188.

All blends obtained were individually mixed with ethanol in a ratio of 12:88. Further all obtained mixtures were individually used in combination with the compositions B as disclosed above.

## Claims

1. Method of moisturizing the mammal skin involving the steps of
a) applying a first cosmetic composition A comprising a polymer polymerized from a monomer mixture comprising
(i) at least one triglycidylether according to one of the formulas, 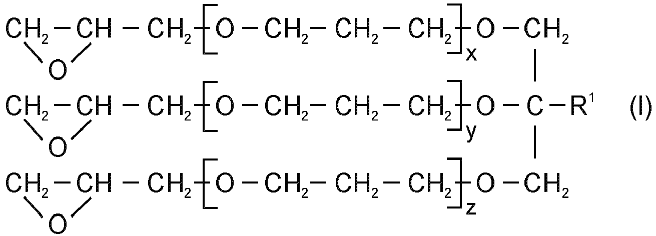 and 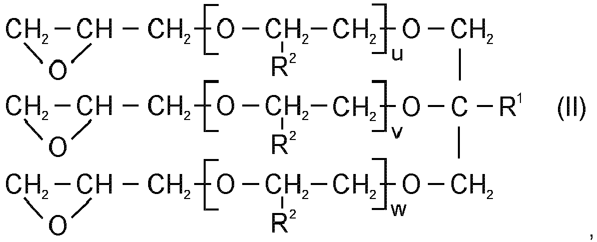 wherein
R¹ stands independently for an alkylgroup having 1 to 4 carbon atoms or a hydrogen atom,
R² stands independently for a linear or branched, saturated or non-saturated hydrocarbon group containing 1 to 18 carbon atoms or a hydrogen atom,
and
u, v, w, x, y and z stand independently for an integer number in the range from 1 to 100;
and
(ii) at least one alkylamine comprising 3 to 30 carbon atoms;
and
b) applying a second cosmetic composition B comprising at least one moisturizer.

2. Method according to claim 1 **characterized in that** the polymer is polymerized from a monomer mixture comprising
(i) 10% to 90% by weight of at least one triglycidylether according to one of the formulas I to II, wherein R¹, R², u, v, w, x, y and z are defined as described in claim 1, and
(ii) 10% to 90% by weight of at least one alkylamine comprising 3 to 30 carbon atoms.

3. Method according to any of the proceeding claims **characterized in that** the polymer is polymerized from a monomer mixture comprising
(i) 50% to 90% by weight of at least one triglycidylether according to one of the formulas I to II, wherein R¹, R², u, v, w, x, y and z are defined as described in claim 1, and
(ii) 10% to 50% by weight of at least one alkylamine comprising 3 to 30 carbon atoms.

4. Method according to any of the proceeding claims **characterized in that** the polymer is polymerized from a monomer mixture comprising
(i) 75% to 88% by weight of at least one triglycidylether according to one of the formulas I to II, wherein R¹, R², u, v, w, x, y and z are defined as described in claim 1, and
(ii) 12% to 25% by weight of at least one alkylamine comprising 3 to 30 carbon atoms.

5. Method according to any of the proceeding claims **characterized in that** the polymer is polymerized from a monomer mixture comprising at least one triglycidylether according to formula I, whereby R¹ is a hydrogen atom and the integer values of x, y and z is less than 20, more preferred less than 10, still more preferred less than 6 and most preferred x, y and z are all equal to 1.

6. Method according to any of the proceeding claims **characterized in that** the polymer is polymerized from a monomer mixture comprising at least one triglycidylether according to formula II, whereby R¹ is a hydrogen atom, R² stands for a methyl group and/or a hydrogen atom, and the integer values of u, v and w is less than 20, more preferred less than 10, still more preferred less than 6 and most preferred u, v and w are all equal to 1.

7. Method according to any of the proceeding claims **characterized in that** the amine is a primary amine.

8. Method according to any of the proceeding claims **characterized in that** the amine comprises a linear alkyl chain comprising 10 to 25 carbon atoms, more preferably 14 to 22 and most preferred 18 carbon atoms.

9. Method according to any of the proceeding claims **characterized in that** the polymer is preferably formed by
a) dissolving the triglycidylether in ethanol,
b) then adding the amine to the solution, and
c) then stirring the obtained mixture to obtain the product.

10. Method according to claim 9 **characterized in that** the reaction product obtained in step c) is further reacted in a further step d) with a nucleophile in order to open the remaining epoxide groups.

11. Method according to claim 10 **characterized in that** the nucleophile is selected from the group of water, alcohols, amines, hydrohalic acids, thiols and/or phosphorous acids.

12. Method according to claim 10 **characterized in that** the nucleophile is selected from the group of water, hydrochloric acid, and alcohols.

13. Method according to any of the proceeding claims **characterized in that** the cosmetic composition A comprises the polymer according to the invention in a total quantity ranging from 0.5 to 30% by weight, more preferably 1 to 20% by weight and most preferably from 5 to 18% by weight, calculated to the total weight of the composition A.

14. Method according to any of the proceeding claims **characterized in that** the cosmetic composition B comprises at least one moisturizer selected from the group of glycerol, propylene glycol and sorbitol, whereby it is preferred if the total quantity of those moisturizers is in the range from 0.1 to 30% by weight, more preferably from 0.5 to 25% by weight and most preferably from 2 to 20% by weight, calculated to the total weight of composition B.

15. Use of a polymer polymerized from a monomer mixture comprising
a) at least one triglycidylether according to one of the formulas I to II, 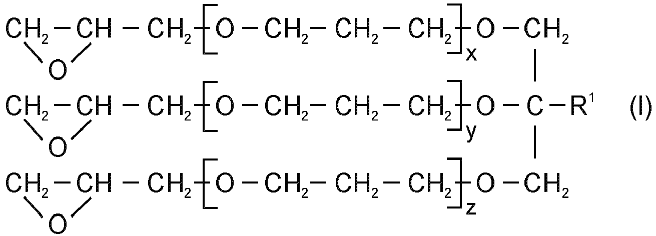 and 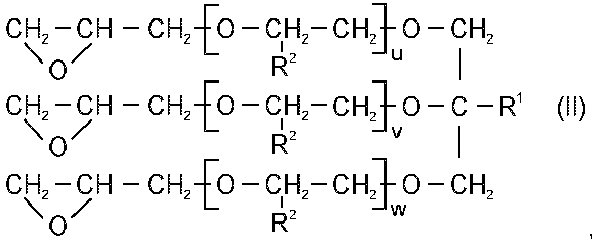 wherein R¹, R², u, v, w, x, y and z are defined as described in claim 1,
and
b) at least one alkylamine comprising 3 to 30 carbon atoms;
to form a coating on the mammal skin, which allows the moisturization of the mammal skin to be increased if a moisturizer is applied onto the coating.
